# EUROPEAN PATENT APPLICATION

(11) **EP 3 760 702 A1**
(43) Date of publication of application: **06.01.2021**
(21) Application number: 19184469.5
(22) Date of filing: 04.07.2019
(51) Int. Cl.: C12M 1/12, C12M 1/00, C12M 3/04

(54) **BIOREACTOR AND BIOREACTOR SYSTEM FOR CELL AND TISSUE GROWTH**

(71) Applicant: Celvivo ApS, 5491 Blommenslyst (DK)
(72) Inventor: WRZESINSKI, Krzysztof Wieslaw, 5491 Blommenslyst (DK); FEY, Stephen John, 5491 Blommenslyst (DK)
(74) Representative: Guardian IP Consulting I/S

(57) **Abstract**

The invention relates to a bioreactor adapted for rotation comprising a vessel which includes: an outer wall and inner wall running along the length direction of the vessel for creating an annular compartment in between the walls, the annular compartment defining a volume for receiving fresh or spent media; an inner chamber defined by the space confined within the inner wall and comprising a fresh media chamber and a spent media chamber; a cell culture chamber in fluid communication the annular compartment and the fresh or spent media chamber, and a movable wall adapted within the inner chamber for separating the fresh media chamber from the spent media chamber. The movable wall is connected to a displacement means in the form of a piston, for displacing the movable wall axially along the length direction of the vessel. The invention relates also to a bioreactor system further comprising driving means for rotating the bioreactor and for displacing the piston.

## Description

The present invention concerns a bioreactor device and bioreactor system for the growing of cell cultures and tissues, in particular a bioreactor operated under omnidirectional normogravity conditions, often incorrectly referred to as microgravity conditions, by continuous rotation of a compartment of the bioreactor containing the cell culture or tissue using a clinostat type device. More particularly, the invention concerns a bioreactor in the form of a vessel comprising a double-wall, where fresh and spent growth media are maintained within the inner chamber of said double wall and are separated by a movable wall, such as a piston. The invention comprises also a bioreactor system including the bioreactor and driving means for displacing the piston and rotating the bioreactor.

Bioreactors for the growing of cell cultures, whether a single or several cell types, or tissues, normally require operation under omnidirectional normogravity conditions i.e. clinostat induced conditions, since this enables the preservation of the differentiated state of many types of cells in the culture or recovery, or (re-)differentiation of in vivo like functionality in cell lines. Such omnidirectional normogravity conditions are induced by continuous rotation of the compartment containing the cell culture, thereby preventing the cells to adhere to the compartment walls (strictly speaking, the rotation infinitesimally increases the gravitational force (centripetal acceleration)). Suitable rotation promotes the adherence of cells to each other in a fluid environment with a minimum of shear forces acting on the culture. Shear forces can be introduced, if needed, for specific cell/tissue types, by changing the rotation speed of the bioreactor. Thereby cells aggregate into colonies typically named spheroids or organoids (in this disclosure referred to collectively as spheroids). Since pieces of tissue will be affected similarly, they are also included under the generic term spheroids.

Stopping the rotation of the bioreactor device is normally needed in order to change the media or add compounds, e.g. drugs or candidate drugs to the compartment, e.g. an incubation chamber or cell culture chamber. However, this results in the problem that during the stop of the rotation, the spheroids settle down to the bottom or adhere to the walls of the compartment. This results in turn in a reduction in the availability of gasses, e.g. oxygen and nutrients to the spheroids thus affecting their quality. Spheroids at the bottom of the sedimented spheroids will suffer more than those at the top. In addition, different users will be more or less skilled in this operation and thus introduce more variability. Thus, stopping the rotation results in changing from an active diffusion environment in a rotating bioreactor to static conditions with limited diffusion rates, resulting in suboptimal growth conditions for spheroid population (and the bigger spheroid size the more severe effect is observed). In particular, this may cause the spheroids to lose important properties such as having a desirable phenotype. It is thus desirable to be able to reduce the number of times that the rotation has to be stopped to a minimum, or ideally never stop its rotation, so as to keep the spheroids as uniform as possible. When the spheroids are very uniform, it is possible to find a particular rotation speed at which they are in a 'stationary orbit' with respect to the media around them and so only tumble very gently in the media solution: this reduces the mechanical shear stress to extremely low levels, i.e. the culture is essentially mechanical 'stress-free'.

Improved uniformity of the spheroids results in a better metabolic performance which then enables for example a more reliable *in vitro* predictive toxicological evaluation of candidate drugs prognosis of the cell culture before going into expensive clinical trials or similar, i.e. it results in a more reliable "filter" prior to embarking on clinical trials.

US 9850458 addresses this challenge by the provision of a lid as the incubation cavity which is fully accessible. However, while the removal of spheroids according to the teaching of the patent is quicker than the previous art, the uniformity of the spheroids is still somewhat impaired, while at the same time the bioreactor requires handling by the operator for enabling the removal of the spheroids.

EP1966367 A1 discloses a bioreactor for incubation of cell cultures, tissue biopsies, cell clusters, tissue-like structures, 'proto-tissues' or similar samples. The bioreactor is adapted for rotation for use in microgravity conditions and equipped with an incubation cavity having a small internal fluid volume, generally less than 1 ml. The small-volume bioreactor permits the use of smaller amounts of reagents which might be of limited supply or might be expensive.

US 2004/0219659 discloses a bioreactor system including components which exert physiologically relevant translational and rotational strains on a growing bioengineered tissue. Thus, physical stress to the cells or tissue is exerted.

US 2013/0230907 discloses a bioreactor designed for generating hydrodynamic pressure, thus subjecting the cell culture to hydrodynamic pressure and thereby exerting physical stress to the cells or tissue.

US 2017/0009207 discloses an apparatus comprising a bioreactor in which the cells therein are exposed to physical stress by means of a mechanical and electrical system. Thus, the apparatus is designed to exert physical stress and/or electrical stimulation to the cells.

US2016/0201037 discloses a bioreactor for growing cells in which the bioreactor device includes a piston. The bioreactor can include an inner body which divides the bioreactor into distinct chambers and facilitates the growth of a multi-tissue sample. The device is designed to grow cells as a layer (or several layers) which are attached to the device. Hence, contact between the device and the cells takes place.

It is therefore an object of the present invention to provide a bioreactor which enables better uniformity of the spheroids, while at the same time avoiding imposing stress on the cell culture during operation as well as more reproducible handling of the bioreactor, i.e. a bioreactor which is easier to operate.

It is another object of the present invention to provide a bioreactor that minimises contact between the walls of the bioreactor and the cells or tissue.

It is yet another object of the present invention to provide a bioreactor which is simple and easy to keep sterile.

It is a further object of the present invention to provide a bioreactor which enables an even suspension i.e. a stationary orbit which reduces shear forces to a minimum of the spheroids being produced therein.

It is a further object of the present invention to provide a bioreactor which enables constant or programmable media supplementation.

It is yet a further object of the present invention to provide a bioreactor which enables preloading of media needed to bioreactor chambers, further minimising bioreactor handling.

These and other objects are solved by the present invention.

Hence, according to a first aspect of the invention, there is provided a bioreactor adapted for rotation, the bioreactor comprising:
a vessel comprising:
a first end and a second end which define a central axis extending along the length direction of the vessel from said first to said second end, an outer wall and inner wall running along the length direction of the vessel for creating an annular compartment in between said walls, said annular compartment defining a volume for receiving fresh or spent media;
an inner chamber defined by the space confined within said inner wall and comprising a fresh media chamber and a spent media chamber;
a cell culture chamber in fluid communication with said annular compartment and said fresh or spent media chamber; and
a movable wall adapted within said inner chamber for separating said fresh media chamber from said spent media chamber, while keeping said fresh and spent media within said inner chamber.

Preferably, said annular compartment defines a volume for receiving fresh media.

Preferably, said culture chamber is in fluid communication with said annular compartment and said spent media chamber.

Accordingly, in an embodiment of the first aspect of the invention, there is provided a bioreactor adapted for rotation, the bioreactor comprising:
a vessel comprising:
a first end and a second end which define a central axis extending along the length direction of the vessel from said first to said second end, an outer wall and inner wall running along the length direction of the vessel for creating an annular compartment in between said walls, said annular compartment defining a volume for receiving fresh media;
an inner chamber defined by the space confined within said inner wall and comprising a fresh media chamber and a spent media chamber;
a cell culture chamber in fluid communication with said annular compartment and said spent media chamber; and
a movable wall adapted within said inner chamber for separating said fresh media chamber from said spent media chamber, while keeping said fresh and spent media within said inner chamber.

The first end of the vessel is preferably flat and vertical. The second end of the vessel is preferably flat and vertical.

Hence, the bioreactor acts as a perfusion bioreactor by which the cell culture in the cell culture chamber is retained and suspended without touching the walls of the vessel, while a source of fresh media providing fresh nutrients is provided and spent media comprising cell waste products is removed. Unlike conventional perfusion bioreactors, the bioreactor of the present invention is simple in its construction and does not require tubing. Furthermore, in contrast to conventional perfusion bioreactors, the bioreactor of the present invention also is adapted for rotation to provide a clinostat cell culture environment.

The bioreactor according to the present invention also allows the media to be exchanged in various ways. For example the media could be provided at a constant speed, or it could be provided three times a day to mimic breakfast, lunch and dinner, or it could be provided to keep a particular component at a predefined level e.g. by adapting into the bioreactor or bioreactor system a component-sensor which activates media exchange.

Gas exchange, e.g. oxygen and carbon dioxide, can occur either through the plastic walls of the vessel, e.g. through polydimethylsiloxane (PDMS) or similar, or through special filters mounted in the walls of the vessel, preferably the walls of the cell culture chamber. Alternatively, the fresh media can be pre-equilibrated before it is filled into the bioreactor.

The inner and outer wall creating the annular compartment thus form a double-wall vessel in which fresh and spent media (growth media) are retained within the inner chamber of the vessel while being separated by the movable wall.

The bioreactor is thus simple, integrated and capable of producing uniform spheroids. The bioreactor is also easier to keep sterile and easier to operate, i.e. by enabling changing or refilling of cell culture over longer periods, e.g. 14 days or more, rather than typically every 48 hrs or so. This time period can be extended by filling the fresh media chamber with a 'stock' solution where one or more of the components that are expected to become exhausted during culture, are present at a higher concentration. For example, the cultures use glucose. Glucose is often provided at a physiological concentration of 1 g/L. If the media in the fresh media chamber contains glucose at 4.5 g/l then the culture can be maintained for 4.5 times the length of time. Media containing both 1 g/L and 4.5 g/L are commercially available from several sources. As an example: for mature C3A spheroids, which are metabolically very active, the media is usually changed every 2 days (other cell types may be different). Considering a cell culture chamber of 10 mL, this means that there would be 7 media changes in 14 days. This would require 70 mL of fresh media. Thus, the media reservoir should be at least 70 mL. If the media contains high levels of glucose (4.5 g/L) then 70 mL would be equivalent to media for 63 days. However, other components in the media may become limiting during this time.

Suitable internal dimensions for a 10 mL cell culture chamber would thus be approximately 3 - 4 cm in diameter and 1.5 - 0.8 cm in height and for the media chamber/reservoir would be approximately 3 - 4 cm in diameter and 10 - 5 cm in height, without taking into account the volume of connecting elements. These can be scaled correspondingly for vessels of other diameters.

Typical flow rates can be calculated from these numbers. For example, if 70 mL of media has to be changed in 14 days at a constant flow rate, the flow rate would be about 0.2 mL per hour. In other instances where the flow should mimic 3 'meals' a day, each taking 30 minutes, the flow rate would be 3.33 ml per hour.

In one embodiment of the invention according to the first aspect, said cell culture chamber is arranged separately at said first end of the vessel and is provided with an inlet orifice for allowing media, e.g. liquid media, from said annular compartment to enter into the cell culture chamber, and an outlet orifice or valve for allowing media, e.g. liquid media, from said culture chamber to enter into said spent media chamber. The movable wall enables fresh media to flow through the annular compartment to the cell culture chamber, while the orifice and particularly a valve prevents the mixing of the two liquids, i.e. the cell culture and the spent media.

The bioreactor of the present invention results also in that the use of filters to separate the cell culture chamber from either the fresh media chamber, the media conduit or the spent media chamber becomes optional. Rotation of the bioreactor will tend to cause the spheroids to move away from the central axis, i.e. away from the outlet port of the cell culture chamber. The influx of media, at even a slow rate, will prevent spheroids from entering the media conduit. Thus the use of a membrane in the bioreactor to prevent spheroids from leaving the cell culture chamber is avoided, thus resulting in a much simpler an inexpensive construction.

Yet, in a particular embodiment of the bioreactor according to the first aspect of the invention, the bioreactor includes a membrane for preventing at least part of the cell culture in the cell culture chamber from exiting said cell culture chamber. Preferably, the membrane is arranged over the inlet and/or outlet orifice of the cell culture chamber. This enables holding components, cells or spheroids in the cell culture chamber and also prevents components or cells, e.g. microorganisms, from entering or exiting the cell chamber. Hence, there may be situations where such a membrane is advantageous. These situations include when microorganisms are cultured, or co-cultured with spheroids which are too small to be significantly influenced either by gravity or by media flow, or they may be motile, or when the user desires to retain compounds preferentially inside or outside of the cell growth chamber.

In another embodiment of the invention according to the first aspect, said annular compartment is provided with an inlet orifice for allowing media (i.e. liquid, or liquid media) from said fresh media chamber to enter into the annular compartment, and an outlet orifice for allowing media from the annular compartment to enter into the cell culture chamber, and wherein said outlet orifice corresponds to said inlet orifice of the cell culture chamber. This facilitates the flow of fresh media into the cell culture chamber. Accordingly, the fresh media chamber is provided with an outlet orifice for allowing media e.g. liquid media from said fresh media chamber to enter into the annular compartment, where this outlet orifice of the annular compartment corresponds to the above inlet orifice of said annular compartment.

In another embodiment of the invention according to the first aspect, at least part of said outer wall is detachable, preferably as a removable end of the outer wall located at said first end of the vessel, for providing access to the cell culture chamber. This enables a much simpler construction of the bioreactor and easier maintenance due to better accessibility to the bioreactor's internal parts, in particular to the cell culture chamber and the cells or spheroids therein. By the term "detachable" is meant that it can be reversibly opened or removed. In a particular embodiment, a flat part of the removable end of the outer wall located at said first end of the vessel includes at least one access port and optionally a sensor, said sensor preferably being mounted in said access port. In another particular embodiment the sensor is mounted in its own location in the chamber wall.

In another embodiment of the invention according to the first aspect, at least part of said outer wall is transparent glass or plastic for permitting observation of the media, cells and spheroids therein. This enables that the cell culture be monitored and assessed from the outside. The glass or plastic, apart from being transparent, is also biologically and chemically inert with the media and cells. Furthermore the plastic should have low affinity to bind, adsorb or absorb any compounds in the media including for example bioactives or candidate drugs. As suitable plastics, polystyrene, polypropylene and polyethylene may be used.

Preferably, the vessel including chambers may be made of glass-clear plastic, while all other components are made of polypropylene.

In another embodiment of the invention according to the first aspect, said inner wall has a plurality of raised ridges extending along the length direction of the vessel, and extending vertically outwards, i.e. radially-perpendicular to said length direction, until contacting said outer wall, and wherein the space between said raised ridges defines one or more sub-compartments within said annular compartment. The raised ridges are preferably arranged in parallel. The raised ridges enable the creation of a better flow of the media in the annular compartment, as this is divided into a number of sub-compartments which better approach a laminar flow condition, thus avoiding back-mixing and turbulent flow that may damage the media flowing therein. The provision of the raised ridges as recited above enables also a simpler construction and thus the manufacturing of the bioreactor in an inexpensive way.

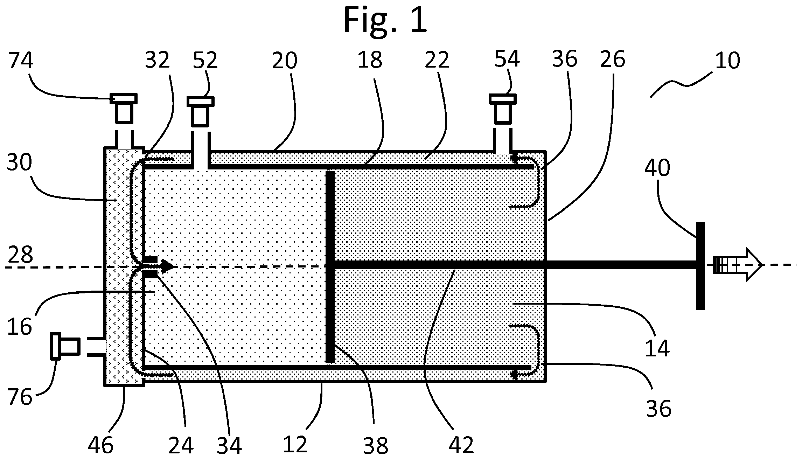

In another embodiment of the invention according to the first aspect, the vessel has a cylindrical shape and is adapted for rotation around a horizontal, rotational axis by associated rotation means, said rotational axis being said central axis running along the length direction of the vessel. The rotation enables the provision of omnidirectional normogravity conditions, meaning that spheroids being formed in the cell culture chamber are suspended in a stable orbit without touching the walls of the vessel. The horizontal axis is as defined by the central axis line 28 in Fig. 1.

In another embodiment of the invention according to the first aspect, said movable wall is connected to a displacement means, for displacing the movable wall axially along the length direction of the vessel, preferably in a direction away from said cell culture chamber. In circumstances where the flow needs to be reversed, the movable wall will be displaced axially along the length direction of the vessel in a direction towards said cell culture chamber. In a particular embodiment, said displacement means is a piston, said movable wall being connected to said piston through a piston shaft which is coincident with said central axis running along the length direction of the vessel. In another particular embodiment, a conduit is provided which runs from the cell culture chamber through the centre of said piston shaft to at least the outside of the vessel. In yet another particular embodiment, the piston shaft is a conduit such as a tube which runs from the cell culture chamber to at least the outside of the vessel. This enables easier and straightforward sampling and monitoring of the cells in the culture chamber. Hence, it permits on-line sampling of media or cells in this chamber without resorting to having to stop the rotation of the bioreactor. Furthermore, from the outside it is possible not only to collect spent media, but also to introduce fresh media, compounds e.g. bioactive molecules, drugs or candidate drugs, or other solutions.

In another embodiment of the invention according to the first aspect, the vessel is rotated at a speed of 0.1-200 rpm. The optimal speed of rotation of the vessel depends on many factors including the age of the spheroids, since as they get older they get larger and require a higher rpm to reach the 'stationary orbit conditions', the temperature and the viscosity of the media used. Fine regulation of the rpm, e.g. to 0.1 rpm, is necessary to reach the 'stationary orbit condition'. Rotation needs to be essentially free of vibration, wow and flutter.

In another embodiment of the invention according to the first aspect, the vessel or part of the vessel is constructed of a gas permeable plastic, or the vessel includes a gas permeable membrane for the exchange of gasses such as oxygen and carbon dioxide. In a particular embodiment, said gas permeable membrane is arranged along said first or second ends of the vessel. In another particular embodiment, said gas permeable membrane is arranged along the circumferential part i.e. the perimeter, of the cell culture chamber, preferably along the circumferential part of said removable end of the outer wall located at said first end of the vessel. Preferably, said gas permeable membrane is a semipermeable membrane.

While the vessel is preferably cylindrically shaped and thereby its perimeter is circular as so is the cell culture chamber arranged therein, the invention also encompasses that the perimeter can be other than circular, such as a polygon.

In another embodiment of the invention according to the first aspect, a humidification system is provided between the culture chamber and the external atmosphere, i.e. the surrounding atmosphere outside the vessel, said humidification system comprising a liquid reservoir preferably containing sterile water, an evaporation chamber such as an evaporation labyrinth and a filter such as a gas permeable membrane, particularly a semipermeable membrane. This enables facilitating the exchange of gasses into the cell culture chamber while simultaneously avoiding the loss of water from the culture chamber.

Preferably, said filter is said gas permeable membrane arranged along the circumferential part i.e. perimeter of the cell culture chamber. In an embodiment, said evaporation chamber e.g. evaporation labyrinth and said liquid reservoir are arranged along the circumferential part of the vessel, in the same manner as the gas-permeable membrane.

In yet another embodiment, the humidification system further comprises an additional filter being arranged in fluid communication with said liquid reservoir and said evaporation chamber, thereby allowing evaporation from the liquid reservoir into the evaporation chamber e.g. evaporation labyrinth. The additional filter is preferably arranged along the circumferential part of the vessel and in between said liquid reservoir and said evaporation chamber. The additional filter is preferably highly permeable to allow evaporation from the liquid reservoir into the evaporation chamber. The additional filter may be a wick or a gas-permeable filter. The latter is most suitable for an embodiment in which the additional filter is arranged along the circumferential part of the vessel. Such gas-permeable filters will then have different properties to the above semipermeable membrane arranged along the circumferential part of the cell chamber.
In a particular embodiment, the humidification system further comprises at least one opening port connecting the evaporation chamber e.g. evaporation labyrinth with the external atmosphere.

In a particular embodiment, a port is provided for allowing air to be sucked into the spent media chamber. This may be necessary, in the event that a negative pressure develops in the vessel.

In another embodiment of the invention according to the first aspect, the cell culture chamber includes at least one access port and optionally a sensor, said sensor preferably being removably mounted in said access port. By being mounted is meant that the sensor is integrated in the access port. This represents an elegant and simple way to continuously measuring the state or conditions of the cell culture. For instance, the sensor mounted in the access port may be adapted to measure the glucose level of the cell culture and if the glucose level is low, activate the piston via a control mechanism so it is pulled out and thus enable the replenishment of the cell culture chamber with fresh media, including fresh glucose.

In another embodiment of the invention according to the first aspect, at least one sensor is mounted on the part of the inner wall of the vessel which is in direct contact with the cell culture chamber.

In another embodiment of the invention according to the first aspect, said outer wall includes an access port to said fresh media chamber. This enables the provision of fresh media in the bioreactor, which is particularly relevant at the beginning of use. This access port is directly connected to the fresh media chamber and is preferably located at the periphery of the outer wall at the second end of the vessel, i.e. farthest away from the cell culture chamber.

In another embodiment of the invention according to the first aspect, said outer wall includes a port for introducing or removing air, liquids, cells, cell aggregates, or biologically active molecules, e.g. drugs. This access port may be the same access port as the above access port to said fresh media chamber. Preferably, the access port is arranged on the circumferential side of the vessel or on the removable part of the outer wall of the vessel.

Any of the access ports or ports has preferably a cup around it to facilitate keeping the region around the access port clean, dry and sterile and facilitate the removal of air bubbles.

In another embodiment of the invention according to the first aspect, an additional cell culture chamber is adapted in series connection with said cell culture chamber, said additional cell culture chamber having a conduit to transfer said fresh media and having an orifice for allowing the media to flow from the cell culture chamber to said additional cell culture chamber. Hence, an extra, preferably petri-dish shaped cell culture is interposed between the removable end of the vessel (i.e. double wall vessel) and the rest of the bioreactor, such an additional cell culture chamber having conduits to transfer the fresh media from the rest of the bioreactor to the removable end of the double walled vessel and having a hole to allow the media to flow between the cell culture chamber and the additional cell culture chamber. In a particular embodiment, further additional cell culture chambers are assembled on the vessel by inserting extra additional cell culture chambers, preferably essentially petri-dish shaped cell culture chambers. In another particular embodiment, at least one sensor is mounted on the one or more additional cell culture chambers in such a way that the contents of each chamber can be monitored independently.

In a second aspect, the invention encompasses also a bioreactor in which the cell culture chamber is in the annular portion of the vessel. Accordingly, there is provided a bioreactor adapted for rotation, the bioreactor comprising:
a vessel including:
a first end and a second end which define a central axis extending along the length direction of the vessel from said first to said second end,
an outer wall and inner wall running along the length direction of the vessel for creating an annular compartment in between said walls;
an inner chamber defined by the space confined within said inner wall and comprising a fresh media chamber and a spent media chamber;
said annular compartment being a cell culture chamber in fluid communication with said spent media chamber and said fresh media chamber;
a movable wall adapted within said inner chamber for separating said fresh media chamber from said spent media chamber, while keeping said fresh and spent media within said inner chamber.

Hence, the cell culture chamber is an annulus, i.e. the cell culture chamber occupies the annular compartment or sub-compartments. This enables a more compact construction of the bioreactor and is well suited for small cell culture chamber volumes. The average radial distance is greater making it easier to find the 'stationary orbit' rotation speed.

In a third aspect, the invention comprises also a bioreactor system for growing a cell culture or tissue, the system comprising:
a bioreactor adapted for rotation, said bioreactor comprising a vessel comprising:
a first end and a second end which define a central axis extending along the length direction of the vessel from said first to said second end, an outer wall and inner wall running along the length direction of the vessel for creating an annular compartment in between said walls, said annular compartment defining a volume for receiving fresh or spent media, preferably fresh media;
an inner chamber defined by the space confined within said inner wall and comprising a fresh media chamber and a spent media chamber;
a cell culture chamber in fluid communication with said annular compartment and said fresh or spent media chamber, preferably said spent media chamber; and
a movable wall adapted within said inner chamber for separating said fresh media chamber from said spent media chamber, while keeping said fresh and spent media within said inner chamber;
wherein said movable wall is connected to a displacement means in the form of a piston, for displacing the movable wall axially along the length direction of the vessel;
the bioreactor system further comprising:
   - retaining rollers adapted for supporting and enabling rotation of the bioreactor;
   - driving means such as drive wheel for rotating the bioreactor;
   - a retaining block for supporting the piston, said piston being connected to said movable wall via a piston shaft;
   - driving means for moving said retaining block and thereby displacing the piston.

Preferably, the driving means for rotating the bioreactor and for moving said retaining block is a syringe pump.

In a fourth aspect, the invention comprises also a bioreactor system for growing a cell culture or tissue, where the cell culture chamber of the bioreactor is in an annulus, i.e. the annular compartment. Accordingly, there is provided a bioreactor system for growing a cell culture or tissue, the system comprising:
a bioreactor adapted for rotation, said bioreactor comprising a vessel including:
   a first end and a second end which define a central axis extending along the length direction of the vessel from said first to said second end, an outer wall and inner wall running along the length direction of the vessel for creating an annular compartment in between said walls;
   an inner chamber defined by the space confined within said inner wall and comprising a fresh media chamber and a spent media chamber;
   said annular compartment being a cell culture chamber in fluid communication with said spent media chamber and said fresh media chamber;
   a movable wall adapted within said inner chamber for separating said fresh media chamber from said spent media chamber, while keeping said fresh and spent media within said inner chamber;
   wherein said movable wall is connected to a displacement means in the form of a piston, for displacing the movable wall axially along the length direction of the vessel;
the bioreactor system further comprising:
   retaining rollers adapted to support and enable rotation of the bioreactor;
   driving means such as a drive wheel for rotating the bioreactor;
   retaining block for supporting the piston, said piston being connected to said movable wall via a piston shaft;
   driving means for moving said retaining block and thereby displacing the piston.

Any of the embodiments of the first aspect may be used together with the second or third or fourth aspect of the invention.

The invention is illustrated by the accompanying drawings, where:
Fig. 1 is an embodiment of the bioreactor according to the first aspect, where the cell culture chamber is at the first flat end of the vessel.
Fig. 2 shows internal parts of the vessel according to the first aspect to illustrate a simple way to construct the bioreactor.
Fig 3 is an embodiment of the bioreactor according to the first aspect, where a humidification system including a filter is provided between the cell culture chamber and the spent media chamber.
Figure 4 is an embodiment of the bioreactor according to the first aspect, where a humidification system including a filter is provided along the circumference of the cell culture chamber, and where the humidification system is provided between the culture chamber and the external atmosphere.
Fig. 5 shows embodiments related to the cell culture chamber 30 and additional cell culture chamber 80.
Fig 6 is an embodiment of the bioreactor according to the second aspect, where the cell culture chamber is an annulus of the vessel.
Fig. 7 shows a bioreactor system according to the third aspect, which includes the bioreactor itself, driving means for rotating the bioreactor and for displacing the piston.

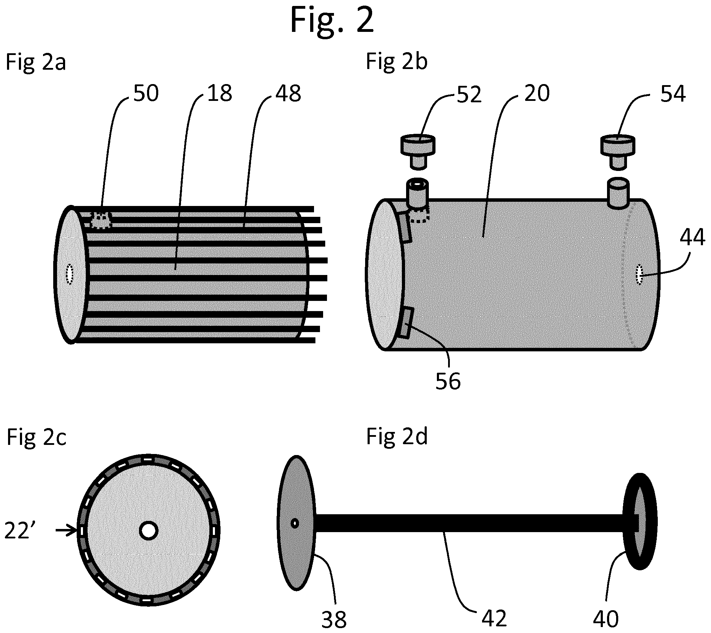

With reference to Fig. 1, the bioreactor 10 comprises a vessel 12 which includes an inner chamber comprising a fresh media chamber 14 and spent media chamber 16. The vessel 12 comprises also an inner wall 18 and outer wall 20, both walls running along the length direction of the vessel 12 for creating an annular compartment 22 in between these walls, and thus forming a double wall vessel. The annular compartment 22 may be one single compartment in the form of a conduit i.e. a media conduit, or a number of sub-compartments or a number of conduits as shown in Fig. 2c. The vessel 12 includes a first end 24, preferably a first flat end and second end 26, preferably second flat end at the opposite side of the vessel 12. The first 24 and second 26 ends define a central axis 28, as shown by the horizontal stippled line, which extends along the length direction of the vessel from said first end 24 to said second end 26. The vessel 12 comprises also a cell culture chamber 30 arranged at said first end 24 of the vessel, as well as a movable wall 38 adapted within the inner chamber for separating the fresh media chamber 14 from said spent media chamber 16, while keeping said fresh and spent media (as shown by dotted areas) within the inner chamber. The cell culture chamber 30 has an inlet orifice 32 for allowing media (liquid media) from the annular compartment 22 to enter into the cell culture chamber 30, as well as an outlet orifice or valve 34 for allowing media (liquid media) from said culture chamber 30 to enter into said spent media chamber 16. Likewise, the annular compartment 22 has an inlet orifice 36 for allowing media from the fresh media chamber 14 to enter into the annular compartment 22, and an outlet orifice 32 for allowing liquid/media from the annular compartment 22 to enter into the cell culture chamber 30. The outlet orifice of the annular compartment corresponds to or is coincident with the above-mentioned inlet orifice 32 of the cell culture chamber 30. The movable wall 38 is connected to the piston 40 via a piston shaft 42. When the piston moves in the right direction, as shown by the arrow, the movable wall 38 is displaced and thereby fresh media from the fresh media chamber 14 passes through the annular compartment 22 into the cell culture chamber 30. The vessel 12 comprises also a removable end 46 of the outer wall 20, which is located at said first end 24 of the vessel.

Fig. 2 shows the internal parts of the bioreactor and illustrates the simple design and construction of the bioreactor by quickly assembling such parts:
Fig. 2a shows the inner cylinder of the vessel, comprising inner wall 18 and a plurality of raised ridges 48 extending along the length direction of the inner wall 18 and thereby vessel 12, and also extending vertically outwards, i.e. perpendicularly to said length direction, in a radial direction, until contacting said outer wall 20. The space between said raised ridges 48 defines one or more sub-compartments 22' within said annular compartment 22, as shown in the side view presented in Fig. 2c. Fig. 2a shows also a hole (orifice) 50 through which an access port 52 (Fig. 2b) reaches spent media chamber 16.

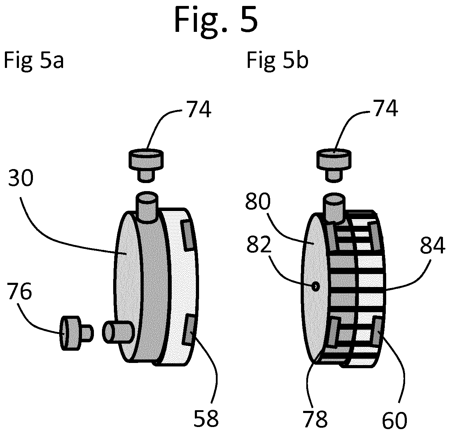

Fig. 2b shows the outer cylinder of the vessel, comprising an outer wall 20. The outer wall 20 is detachable and comprises access port 52 which is adapted to cooperate with hole 50 of the inner wall 18 (Fig. 2a). The access port 52 serves also as a pressure equalization port, e.g. for relieving pressure differences. The outer wall 20 includes access port 54 (and stopper) for allowing the introduction of fresh media into fresh media chamber 14. A locking flange 56 is also provided, and which engages with corresponding flange 58 or 60 (Fig. 5a or b).

Fig. 2c is a cross-section when the inner cylinder is fitted into the outer cylinder. The raised ridges 48 arranged in parallel create a number of annular sub-compartments or conduits 22'.

Fig. 2d shows the internal part of the vessel in the form of a movable wall 38 which is connected to piston 40 via piston shaft 42. When the bioreactor is assembled, the piston shaft 42 passes through orifice 44 in the flat end 26 of the outer wall.

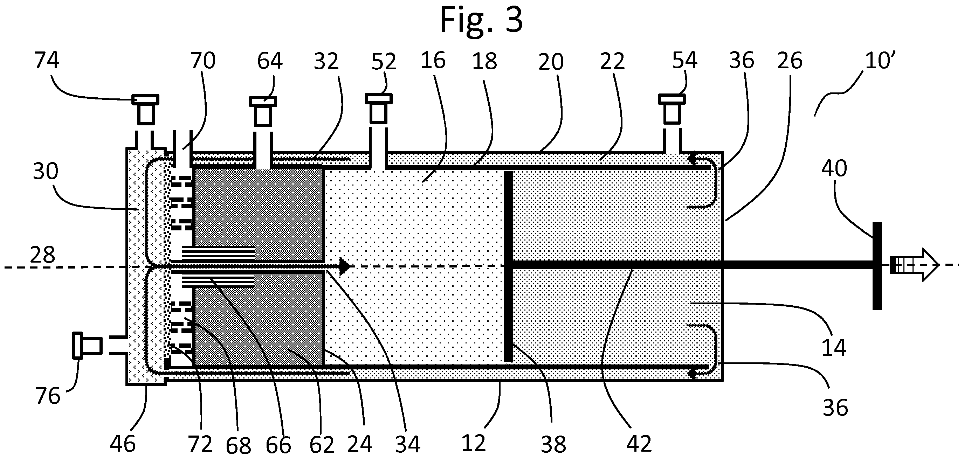

Fig 3 shows an embodiment of the bioreactor according to the first aspect, where a humidification system is inserted between the cell culture chamber and the spent media chamber. The humidification system, i.e. humidifier, comprises a liquid reservoir 62, evaporation chamber such as an evaporation labyrinth 68 and filter 72. A liquid, typically sterile water, would be filled into the liquid reservoir 62 via port 64. This liquid would be conveyed via wick 66 to the evaporation labyrinth 68 where it would humidify the atmosphere. The evaporation labyrinth 68 has at least one open port 70 to the atmosphere surrounding the equipment by which gasses, e.g. oxygen and carbon dioxide, can be exchanged. The humidified atmosphere is in direct contact with a filter such as semipermeable membrane 72, which forms one of the walls of the culture chamber 30. By this means, gasses can be relatively freely exchanged between the culture chamber 30 and the atmosphere around the equipment without a net evaporation of liquids from the culture chamber 30. In practice water is lost from the liquid reservoir 62 at a very approximate rate of 1 - 2 mL per week. Consequently, the reservoir 62 needs to have a volume of 10 - 20 mL for a 10 mL bioreactor as described here. The filter 72, evaporation labyrinth 68 and liquid reservoir 62 have a conduit which connects the cell culture chamber with the spent media chamber.

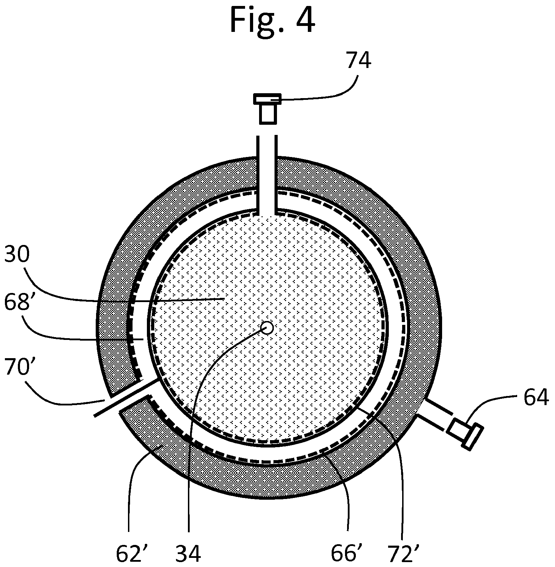

Fig. 4 shows an embodiment of the bioreactor according to the first aspect, where the humidification system includes a filter in the form of a semipermeable membrane 72', which is arranged circumferentially around the cell culture chamber 30, i.e. along the circumferential part of the cell culture chamber. The humidification system includes said filter 72', liquid reservoir 62' containing sterile water, additional filter such as wick or gas permeable filter 66' evaporation chamber in the form of evaporation labyrinth 68'. The additional filter 66' would be highly permeable to allow evaporation from the liquid reservoir 62' into the evaporation labyrinth 68'. Hence, in this embodiment, it will be simpler to replace the wick 66 of the embodiment of Fig. 3 with a gas-permeable filter 66', which will then have different properties to the semipermeable membrane 72'. The humidification system is also provided with port 64 for refilling of liquid reservoir 62' and at least one open port 70' connecting the evaporation labyrinth 68' with the external atmosphere. For access to the cell culture chamber 30 of the bioreactor, port 74 is arranged as already described in e.g. Fig 1.

The embodiment of the bioreactor shown in Fig. 4 is preferential to that shown in Fig.1 because the presence of the humidification system will greatly enhance the gas exchange between the cell culture chamber 30 (or additional culture chamber(s) 80) and the surrounding atmosphere and will reduce or eliminate water loss from said cell culture chambers 30 or 80. The difference is so significant that the bioreactor will normally be able to be used in an incubator without additional humidification. This is a great advantage because it will reduce the risk of infection in the incubator.

The embodiment of the bioreactor shown in Fig. 4 is also preferential to that shown in Fig. 3 for at least three reasons. The first is that it will be easier to regulate the gas exchange by having a stopper or sliding cover which can partially or completely close port 70' so that it is possible to regulate gas exchange of an individual bioreactor, rather than having to regulate gas atmosphere of the whole incubator. The second reason is that the bioreactor illustrated is more compact along the axis 28 and finally is less expensive to manufacture.

Fig. 5a shows the cell culture chamber 30 including circumferential access port 74 and the planar access port 76. These ports provide access to the cell culture of the cell culture chamber 30 and may be provided with a sensor for monitoring the conditions of the cell culture, for instance the glucose level. A locking flange 58 engaging with a corresponding flange 56 (Fig. 2b) or 78 (Fig. 5b) is also provided.

Fig. 5b shows an additional cell culture chamber 80 that can be inserted between the first cell culture chamber 30 and the double walled vessel containing the spent and fresh growth media. The additional cell culture chamber 80 contains an orifice or hole 82 for allowing media, e.g. liquid media, to flow between the two cell culture chambers 30 and 80.

Accordingly, these cell culture chambers may be regarded as being arranged in series. A conduit 84 is also arranged, which allows liquid flow between fresh media chamber 14, through conduit 22 and via (without mixing) at least one additional cell culture chamber 80 to the cell culture chamber 30. Thus, further additional cell culture chambers may be arranged therein.

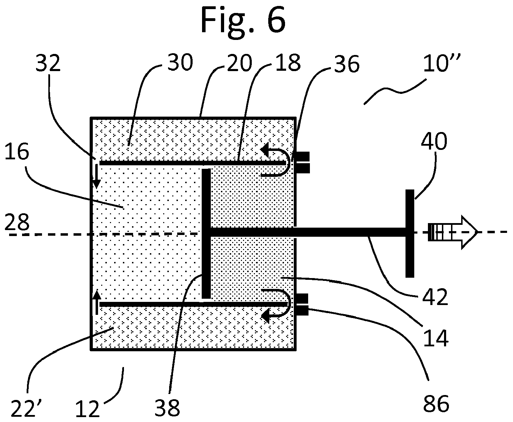

Fig 6 is an embodiment of the bioreactor 10' according to the second aspect of the invention, where the cell culture chamber 30 is an annulus of the vessel 12. Hence, the annular compartment 22, 22' is the cell culture chamber 30 and which is in fluid communication with said spent media chamber 16 and said fresh media chamber 14. A more compact construction results. The vessel 12 is preferably provided with an arrangement 86 for opening and closing the bioreactor. Access ports, sensor locations and humidification systems have been omitted for clarity but can of course be included in a similar manner as that described above.

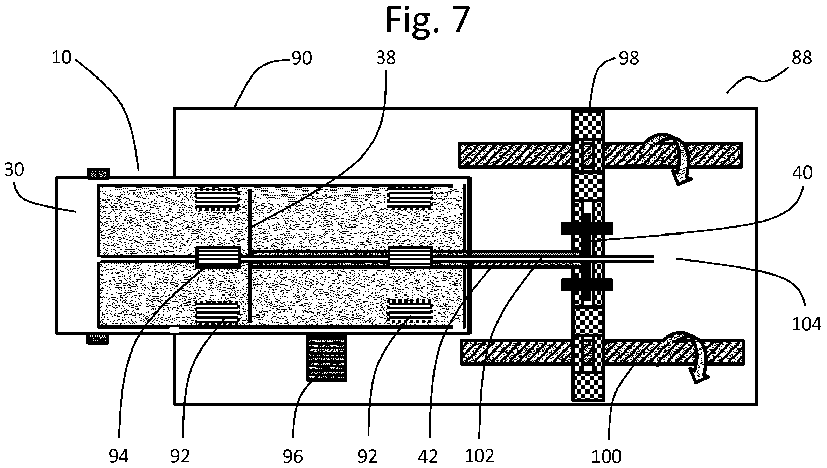

Fig. 7 illustrates a bioreactor system 88 according to the third aspect of the invention. The system may be mounted on a platform 90 and comprises the bioreactor 10 comprising cell culture chamber 30 at a first flat end of the double wall vessel 12. Instead of the bioreactor embodiment 10 exemplified in Fig. 1-3, the bioreactor embodiment 10' exemplified in Fig. 4 may also be used. Retaining rollers 92 and 94 are provided respectively underneath and on top of the vessel 12 to support and enable rotation of the bioreactor 10. Driving means in the form of a drive wheel 96 are affixed the vessel 12 and thus the bioreactor 10, thereby providing the rotation. This function may also be carried out by retaining rollers 92 and/or 94. The system comprises also a retaining block 98 for supporting the piston 40 which includes piston shaft 42 and movable wall 38, and drive means 100 to move the retaining block 98. An optional conduit 102, such as a tube, is provided, which runs through the central axis of piston e.g. inside the piston shaft 42 to the outside 104. Direct and simple sampling of the cells from the cell culture chamber 30 is thus obtained.

### List of parts

10. Bioreactor adapted for rotation
12. Vessel
14. Fresh media chamber
16. Spent media chamber
18. Inner wall of vessel
20. Outer wall of vessel
22. Annular compartment or sub-compartment. Media conduit
24. First (flat) end of vessel
26. Second (flat) end of vessel
28. Central axis extending along the length direction of the vessel
30. Cell culture chamber
32. Orifice allowing media (liquid media) to flow from 22 to 30
34. Orifice or valve
36. Orifice allowing media (liquid media) to flow from 14 to 22
38. Movable wall
40. Piston
42. Piston shaft
44. Orifice for piston shaft
46. Removable end of outer wall 20
48. Raised ridges in inner wall 18
50. Hole (orifice) through which access port 52 reaches 16
52. Access port. Pressure equalization port
54. Access port to fresh media chamber 14
56. Locking flange for 58 or 60
58. Locking flange to engage with 56 or 78
60. Locking flange for 56 (or 78)
62. Liquid reservoir
64. Port for refilling liquid reservoir 62
66. Additional filter, e.g. wick for transporting and evaporating liquid from reservoir 62 into evaporation chamber 68
68. Evaporation chamber / Evaporation labyrinth
70. Open port connecting the evaporation labyrinth 68 with the external atmosphere
72. Filter e.g. semipermeable membrane in direct contact with the air in the evaporation labyrinth 68 and with the liquid in the culture chamber 30 or 80
74. Access port to cell culture chamber 30 or 80 (circumferential)
76. Access port to cell culture chamber 30 (planar)
78. Locking flange for 58 (or 60)
80. Additional cell culture chamber
82. Orifice (hole) allowing liquid media to flow between 30 and 80 (or between 80 and another 80)
84. Conduit allowing liquid media flow between 22 and cell culture chamber 30
86. Arrangement for opening and closing the bioreactor
88. Bioreactor system including bioreactor and piston drive system
90. Platform
92. Retaining roller underneath vessel (underneath cylinders)
94. Retaining roller on top of vessel (on top of cylinders)
96. Drive wheel to rotate bioreactor
98. Piston retaining block
100. Drive to move piston retaining block
102. Conduit from cell culture chamber 30, through central axis of piston shaft to outside
104. Outside

It should further be noted that a prime (') or double prime (") symbols in the description and in the figures denote alternative realizations of the same part.

### Points of the invention

1. A bioreactor adapted for rotation, the bioreactor comprising:
   a vessel comprising:
   a first end and a second end which define a central axis extending along the length direction of the vessel from said first to said second end, an outer wall and inner wall running along the length direction of the vessel for creating an annular compartment in between said walls, said annular compartment defining a volume for receiving fresh or spent media, preferably fresh media;
   an inner chamber defined by the space confined within said inner wall and comprising a fresh media chamber and a spent media chamber;
   a cell culture chamber in fluid communication with said annular compartment and said fresh or spent media chamber, preferably said spent media chamber; and
   a movable wall adapted within said inner chamber for separating said fresh media chamber from said spent media chamber, while keeping said fresh and spent media within said inner chamber.
2. A bioreactor according to point 1, wherein said cell culture chamber is arranged separately at said first end of the vessel and is provided with an inlet orifice for allowing media from said annular compartment to enter into the cell culture chamber, and an outlet orifice or valve for allowing media from said culture chamber to enter into said spent media chamber.
3. A bioreactor according to point 1 or 2, wherein said annular compartment is provided with an inlet orifice for allowing media from said fresh media chamber to enter into the annular compartment, and an outlet orifice for allowing media from the annular compartment to enter into the cell culture chamber, and wherein said outlet orifice of the annular compartment corresponds to said inlet orifice of the cell culture chamber.
4. A bioreactor according to any of points 1-3, wherein at least part of said outer wall is detachable, preferably as a removable end of the outer wall located at said first end of the vessel, for providing access to the cell culture chamber.
5. A bioreactor according to any of points 1-4, wherein at least part of said outer wall is transparent glass or plastic for permitting observation of the media, cells and spheroids therein.
6. A bioreactor according to any of points 1-5, wherein said inner wall has a plurality of raised ridges extending along the length direction of the vessel, and extending vertically outwards until contacting said outer wall, and wherein the space between said raised ridges define one or more sub-compartments within said annular compartment.
7. A bioreactor according to any of points 1-6, wherein the vessel has a cylindrical shape and is adapted for rotation around a horizontal, rotational axis by associated rotation means, said rotational axis being said central axis running along the length direction of the vessel.
8. A bioreactor according to any of points 1-7, wherein said movable wall is connected to a displacement means for displacing the movable wall axially along the length direction of the vessel.
9. A bioreactor according to point 8, wherein said displacement means is a piston, said movable wall being connected to said piston through a piston shaft which is coincident with said central axis running along the length direction of the vessel.
10. A bioreactor according to point 9, wherein a conduit is provided which runs from the cell culture chamber through the centre of said piston shaft to at least the outside of the vessel.
11. A bioreactor according to any of points 1-10, wherein the vessel is rotated at a speed of 0.1-200 rpm.
12. A bioreactor according to any of points 1-11, wherein the vessel or part of the vessel is constructed of a gas permeable plastic,
   or
   the vessel includes a gas permeable membrane for the exchange of gasses such as oxygen and carbon dioxide, said gas permeable membrane being arranged along the circumferential part of the cell culture chamber.
13. A bioreactor according to point 12, wherein a humidification system is provided between the culture chamber and the external atmosphere, said humidification system comprising a liquid reservoir preferably containing sterile water, an evaporation chamber such as an evaporation labyrinth and a filter.
14. A bioreactor according to claim 13, wherein said filter is said gas permeable membrane being arranged along the circumferential part i.e. perimeter of the cell culture chamber, said gas permeable membrane preferably being a semipermeable membrane.
15. A bioreactor according to claim 13 or 14, wherein said humidification system further comprises and additional filter being arranged in fluid communication with said liquid reservoir and said evaporation chamber,
16. A bioreactor according to claim 15, said additional filter being arranged along the circumferential part of the vessel and in between said liquid reservoir and said evaporation chamber.
17. A bioreactor according to any of claims 1-16, wherein the cell culture chamber includes at least one access port and optionally a sensor, said sensor preferably being removably mounted in said access port.
18. A bioreactor according to any of points 1-17, wherein said outer wall includes an access port to said fresh media chamber.
19. A bioreactor according to any of points 1-18, wherein an additional cell culture chamber is adapted in series connection with said cell culture chamber, said additional cell culture chamber having a conduit to transfer said fresh media and having an orifice for allowing the media to flow from the cell culture chamber to said additional cell culture chamber.
20. A bioreactor adapted for rotation, the bioreactor comprising:
   a vessel including:
   a first end and a second end which define a central axis extending along the length direction of the vessel from said first to said second end,
   an outer wall and inner wall running along the length direction of the vessel for creating an annular compartment in between said walls;
   an inner chamber defined by the space confined within said inner wall and comprising a fresh media chamber and a spent media chamber;
   said annular compartment being a cell culture chamber in fluid communication with said spent media chamber and said fresh media chamber;
   a movable wall adapted within said inner chamber for separating said fresh media chamber from said spent media chamber, while keeping said fresh and spent media within said inner chamber.
21. A bioreactor system for growing a cell culture or tissue, the system comprising:
   a bioreactor adapted for rotation, said bioreactor comprising a vessel comprising:
      a first end and a second end which define a central axis extending along the length direction of the vessel from said first to said second end, an outer wall and inner wall running along the length direction of the vessel for creating an annular compartment in between said walls, said annular compartment defining a volume for receiving fresh or spent media, preferably fresh media;
      an inner chamber defined by the space confined within said inner wall and comprising a fresh media chamber and a spent media chamber;
      a cell culture chamber in fluid communication with said annular compartment and said fresh or spent media chamber, preferably said spent media chamber; and
      a movable wall adapted within said inner chamber for separating said fresh media chamber from said spent media chamber, while keeping said fresh and spent media within said inner chamber;
   wherein said movable wall is connected to a displacement means in the form of a piston, for displacing the movable wall axially along the length direction of the vessel;
   the bioreactor system further comprising:
      retaining rollers adapted for supporting and enabling rotation of the bioreactor;
      driving means such as a drive wheel for rotating the bioreactor;
      a retaining block for supporting the piston, said piston being connected to said movable wall via a piston shaft;
      driving means for moving said retaining block and thereby displacing the piston.
22. A bioreactor system for growing a cell culture or tissue, the system comprising:
   a bioreactor adapted for rotation, said bioreactor comprising a vessel including:
      a first end and a second end which define a central axis extending along the length direction of the vessel from said first to said second end, an outer wall and inner wall running along the length direction of the vessel for creating an annular compartment in between said walls;
      an inner chamber defined by the space confined within said inner wall and comprising a fresh media chamber and a spent media chamber;
      said annular compartment being a cell culture chamber in fluid communication with said spent media chamber and said fresh media chamber;
      a movable wall adapted within said inner chamber for separating said fresh media chamber from said spent media chamber, while keeping said fresh and spent media within said inner chamber;
   wherein said movable wall is connected to a displacement means in the form of a piston, for displacing the movable wall axially along the length direction of the vessel;
   the bioreactor system further comprising:
      retaining rollers adapted to support and enable rotation of the bioreactor;
      driving means such as drive wheel for rotating the bioreactor;
      retaining block for supporting the piston, said piston being connected to said movable wall via a piston shaft;
      driving means for moving said retaining block and thereby displacing the piston.
23. A bioreactor according to any of points 1-19, wherein the bioreactor includes a membrane for preventing at least part of the cell culture in the cell culture chamber from exiting said cell culture chamber.
24. A bioreactor according to point 23, wherein the membrane is arranged over the inlet and/or outlet orifice of the cell culture chamber.
25. A bioreactor according to point 9, wherein the piston shaft is a conduit such as a tube which runs from the cell culture chamber to at least the outside of the vessel.
26. A bioreactor according to any of points 1-19, wherein further additional cell culture chambers are assembled on the vessel by inserting extra additional cell culture chambers, preferably essentially petri-dish shaped cell culture chambers.
27. A bioreactor according to point 26, wherein at least one sensor is mounted on the one or more additional cell culture chambers in such a way that the contents of each chamber can be monitored independently.
28. A bioreactor according to point 20, comprising any of the features of points 1-19.
29. A bioreactor according to point 21, comprising any of the features of points 1-19.
30. A bioreactor according to point 22, comprising any of the features of points 1-19.

## Claims

1. A bioreactor adapted for rotation, the bioreactor comprising:
a vessel comprising:
a first end and a second end which define a central axis extending along the length direction of the vessel from said first to said second end, an outer wall and inner wall running along the length direction of the vessel for creating an annular compartment in between said walls, said annular compartment defining a volume for receiving fresh or spent media;
an inner chamber defined by the space confined within said inner wall and comprising a fresh media chamber and a spent media chamber;
a cell culture chamber in fluid communication with said annular compartment and said fresh or spent media chamber; and
a movable wall adapted within said inner chamber for separating said fresh media chamber from said spent media chamber, while keeping said fresh and spent media within said inner chamber.

2. A bioreactor according to claim 1, wherein said cell culture chamber is arranged separately at said first end of the vessel and is provided with an inlet orifice for allowing media from said annular compartment to enter into the cell culture chamber, and an outlet orifice or valve for allowing media from said culture chamber to enter into said spent media chamber.

3. A bioreactor according to claim 1 or 2, wherein said annular compartment is provided with an inlet orifice for allowing media from said fresh media chamber to enter into the annular compartment, and an outlet orifice for allowing media from the annular compartment to enter into the cell culture chamber, and wherein said outlet orifice of the annular compartment corresponds to said inlet orifice of the cell culture chamber.

4. A bioreactor according to any of claims 1-3, wherein at least part of said outer wall is detachable, preferably as a removable end of the outer wall located at said first end of the vessel, for providing access to the cell culture chamber.

5. A bioreactor according to any of claims 1-4, wherein at least part of said outer wall is transparent glass or plastic for permitting observation of the media, cells and spheroids therein.

6. A bioreactor according to any of claims 1-5, wherein said inner wall has a plurality of raised ridges extending along the length direction of the vessel, and extending vertically outwards until contacting said outer wall, and wherein the space between said raised ridges define one or more sub-compartments within said annular compartment.

7. A bioreactor according to any of claims 1-6, wherein the vessel has a cylindrical shape and is adapted for rotation around a horizontal, rotational axis by associated rotation means, said rotational axis being said central axis running along the length direction of the vessel.

8. A bioreactor according to any of claims 1-7, wherein said movable wall is connected to a displacement means for displacing the movable wall axially along the length direction of the vessel.

9. A bioreactor according to claim 8, wherein said displacement means is a piston, said movable wall being connected to said piston through a piston shaft which is coincident with said central axis running along the length direction of the vessel.

10. A bioreactor according to claim 9, wherein a conduit is provided which runs from the cell culture chamber through the centre of said piston shaft to at least the outside of the vessel.

11. A bioreactor according to any of claims 1-10, wherein the vessel or part of the vessel is constructed of a gas permeable plastic,
or
the vessel includes a gas permeable membrane for the exchange of gasses such as oxygen and carbon dioxide, said gas permeable membrane being arranged along the circumferential part of the cell culture chamber.

12. A bioreactor according to claim 11, wherein a humidification system is provided between the culture chamber and the external atmosphere, said humidification system comprising a liquid reservoir preferably containing sterile water, an evaporation chamber such as an evaporation labyrinth and a filter.

13. A bioreactor according to claim 12, wherein said filter is said gas permeable membrane being arranged along the circumferential part of the cell culture chamber.

14. A bioreactor according to any of claims 1-13, wherein the cell culture chamber includes at least one access port and optionally a sensor, said sensor preferably being removably mounted in said access port.

15. A bioreactor according to any of claims 1-14, wherein said outer wall includes an access port to said fresh media chamber.

16. A bioreactor according to any of claims 1-15, wherein an additional cell culture chamber is adapted in series connection with said cell culture chamber, said additional cell culture chamber having a conduit to transfer said fresh media and having an orifice for allowing the media to flow from the cell culture chamber to said additional cell culture chamber.

17. A bioreactor adapted for rotation, the bioreactor comprising:
a vessel including:
a first end and a second end which define a central axis extending along the length direction of the vessel from said first to said second end,
an outer wall and inner wall running along the length direction of the vessel for creating an annular compartment in between said walls;
an inner chamber defined by the space confined within said inner wall and comprising a fresh media chamber and a spent media chamber;
said annular compartment being a cell culture chamber in fluid communication with said spent media chamber and said fresh media chamber;
a movable wall adapted within said inner chamber for separating said fresh media chamber from said spent media chamber, while keeping said fresh and spent media within said inner chamber.

18. A bioreactor system for growing a cell culture or tissue, the system comprising:
a bioreactor adapted for rotation, said bioreactor comprising a vessel comprising:
a first end and a second end which define a central axis extending along the length direction of the vessel from said first to said second end, an outer wall and inner wall running along the length direction of the vessel for creating an annular compartment in between said walls, said annular compartment defining a volume for receiving fresh or spent media, preferably fresh media;
an inner chamber defined by the space confined within said inner wall and comprising a fresh media chamber and a spent media chamber;
a cell culture chamber in fluid communication with said annular compartment and said fresh or spent media chamber, preferably said spent media chamber; and
a movable wall adapted within said inner chamber for separating said fresh media chamber from said spent media chamber, while keeping said fresh and spent media within said inner chamber;
wherein said movable wall is connected to a displacement means in the form of a piston, for displacing the movable wall axially along the length direction of the vessel;
the bioreactor system further comprising:
retaining rollers adapted for supporting and enabling rotation of the bioreactor;
driving means such as a drive wheel for rotating the bioreactor;
a retaining block for supporting the piston, said piston being connected to said movable wall via a piston shaft;
driving means for moving said retaining block and thereby displacing the piston.
